# EUROPEAN PATENT APPLICATION

(11) **EP 2 397 857 A1**
(43) Date of publication of application: **21.12.2011**
(21) Application number: 10741227.2
(22) Date of filing: 09.02.2010
(51) Int. Cl.: G01N 33/579, G01N 21/82, G01N 33/483

(54) **METHOD FOR ASSAYING PHYSIOLOGICAL SUBSTANCE OF BIOLOGICAL ORIGIN AND ASSAY DEVICE THEREFOR**

(30) Priority: 13.02.2009 JP 2009031852
(71) Applicant: Kowa Company, Ltd., Nagoya-shi, Aichi 460-8625 (JP)
(72) Inventor: HIRONO, Taisuke, Hamamatsu-shi Shizuoka 431-2103 (JP)
(74) Representative: Elsy, David
(86) International application number: PCT/JP2010/051883
(87) International publication number: WO 2010/092950

(57) **Abstract**

Provided is an assay method whereby assay time can be largely shortened in the case of detecting a physiologically active substance of biological origin such as an endotoxin or β-D-glucan or measuring the concentration of the same with the use of a reaction between said physiologically active substance and LAL. Also provided is an assay device using the same. The aggregation start time of a liquid mixture of an assay sample, which is to be subjected to the detection of a physiologically active substance or the measurement of the concentration of the same, with LAL is determined. From this aggregation start time, the physiologically active substance is detected or the concentration thereof is measured. The liquid mixture of the assay sample with LAL is stirred with, for example, a magnetic stirrer toformgelparticles.Next,thescatteredlightintensity of laser light scattered by these gel particles is measured. Then, the frequency distribution of the scattered light intensity fluctuation is obtained. Based on the temporal change in the frequency distribution shape, the aggregation start time of the liquid mixture of the assay sample with LAL is determined

## Description

### TECHNICAL FIELD

The present invention relates to a measurement method and a measurement apparatus for detecting a biologically active substance in a sample or measuring the concentration of the biologically active substance, where the sample contains the biogenous biologically active substance, such as endotoxin or β-D-glucan, having a characteristic feature of gelation caused by a reaction with a limulus hemocyte extract.

### BACKGROUND ART

Endotoxin is a lipopolysaccharide existing in cell walls of gram-negative bacteria and are the most typical pyrogenic substances. When an infusion solution, an injectable agent, the blood, or the like contaminated with endotoxin enters the human body, serious side effects, such as fever and shock, may be caused. Thus, it is obliged to manage the above medicines or the like to prevent them from being contaminated with endotoxin.

By the way, a serine protease, which can be activated by endotoxin, is present in an extract of limulus hemocytes (also referred to as "LAL: Limulus amoebocyte lysate"). When LAL reacts with endotoxin, an enzyme cascade with serine protease, which is activated depending on the amount of endotoxin, causes hydrolyzation of coagulogens in LAL into coagulins. Then, coagulins are associated together, generating an insoluble gel. This characteristic feature of LAL may be used for detecting endotoxin at a high sensitivity.

Furthermore, β-D-glucan is a polysaccharide which constitutes a cell membrane characteristic of fungus. The measurement of β-D-glucan is effective for screening for a wide range of fungal infectious diseases not only including common clinical fungus, such as Candida, Spergillus, and Cryptococcus, but also including uncommon fungus.

Likewise, in measurement of β-D-glucan, it is possible to detect β-D-glucan at high sensitivity using the property of a limulus hemocyte extract that it can be solidified (gel solidified) by β-D-glucan.

As a method for detecting the presence of or measuring the concentration of a biogenous biologically active substance (hereinafter, also referred to as a predetermined biologically active substance) such as endotoxin and β-D-glucan by a limulus hemocyte extract, the following methods can be exemplified. That is, for example, there are a semi-quantitative gelation method, a turbidimetric method, and a colorimetric method. First, the semi-quantitative gelationmethod is provided for investigating the presence or absence of endotoxin of at least a predetermined concentration. The method includes leaving a liquid mixture standing, where the liquid mixture is prepared by mixing LAL with a sample to be subjected to detection of a predetermined biologically active substance or concentration measurement thereof (hereinafter, simply referred to as "measurement of a predetermined biologically active substance"), turning a vessel upside down after a predetermined time, observing the presence or absence of the sample being dropped to determine whether the sample turns into a gel. The turbidimetric method is provided for temporally measuring the turbidity of a sample accompanying generation of a gel by the reaction of a predetermined biologically active substance with LAL. The colorimetric method uses a synthetic substrate that generates color when it is hydrolyzed by an enzyme cascade.

In the case of measuring the predetermined biologically active substance with the above turbidimetric method, a liquidmixture of the measurement sample and the LAL is generated in a dry-sterilized measurement glass cell. Then, the gelation of the liquid mixture is optically measured from the outside. In some cases, however, the turbidimetric method may take a very long time for gelation of LAL particularly in a sample with a low concentration of the predetermined biologically active substance. On the other hand, a method for allowing a predetermined biologically active substance to be measured within a short time has been desired. As a method that allows a short-time measurement, a light scattering method or a stirring turbidimetric method has been proposed. The light scattering method (laser light scattering particle counting method) is able to measure the presence of a predetermined biologically active substance in a sample within a short time by stirring a liquid mixture of a measurement sample and LAL by a magnetic stirring bar to generate gel particles. Then, the presence of the predetermined biologically active substance in the sample can be determined within a short time from the intensity of laser light scattered by gel particles or the intensity of light passing through the liquid mixture.

Various methods as described above intend to shorten the detection or measurement time of a predetermined biologically active substance and improve the sensitivity of measurement. However, any of these methods has both its advantage and disadvantage. Thus, further improvement has been desired for shortening a measurement time, increasing sensitivity, excluding an interfering substance, and so on.

### CITATION LIST

### PATENT DOCUMENT

Patent Document 1: Japanese Patent Application Laid-Open No. 2004-061314
Patent Document 2: Japanese Patent Application Laid-Open No. 10-293129
Patent document 3: PCT International Publication No. WO 2008/038329

### SUMMARY OF THE INVENTION

### PROBLEM TO BE SOLVED BY THE INVENTION

The present invention has been made in consideration of the aforementioned problems and an object of the invention is to provide a measuring method whose measurement accuracy is high or which can shorten a measurement time, and a measurement apparatus using the method in detection or concentration measurement of a biogenous biologically active substance.

### MEANS FOR SOLVING THE PROBLEM

In the present invention, an aggregation-starting time for a liquid mixture of LAL and a measurement sample, which is a target for detection of a predetermined biologically active substance or concentration measurement thereof, is detected and the detection of the predetermined biologically active substance or the concentration measurement thereof is performed with reference to the aggregation-starting time. The greatest characteristic feature of the present invention is as follows. A measurement sample is mixed with LAL to generate gel particles. Then, the scattered light intensity of laser light which is scattered from the gel particles is measured. Subsequently, a frequency distribution of fluctuations of the scattered light intensity is acquired. Based on a change in the frequency distribution profile over time, an aggregation-starting time for the liquid mixture of the measurement sample and LAL is detected.

More specifically, there is provided a method for measuring a biogenous biologically active substance, including reacting a biogenous biologically active substance in a sample with a limulus amoebocyte lysate, LAL, and detecting the biologically active substance in the sample or measuring the concentration of the biologically active substance, the method including:
after mixing the sample and LAL, making light incident on a liquid mixture of the sample and LAL;
obtaining an intensity of scattered light of the incident light from the liquid mixture;
detecting an aggregation-starting time in the liquid mixture based on a temporal change of frequency distribution of fluctuations of the intensity of the scattered light; and
detecting the biologically active substance in the sample or measuring the concentration of the biologically active substance in the sample by means of the aggregation-starting time.

In the present invention, a dynamic light-scattering technology is applied to a reaction system of a predetermined biologically active substance and LAL. This dynamic light-scattering technology regards the generation and growth of gel particles in a liquid mixture of LAL and a sample containing a predetermined biologically active substance as a change in frequency distribution of fluctuations of the intensity of light scattered from the liquid mixture. Then, an aggregation-starting time in a reaction system is detected based on a change in frequency distribution over time.

Here, in the reaction system of a predetermined biologically active substance and LAL, the frequency of thermal movements of gel particles and movement due to an external force become small as the particle size becomes large. Therefore, the frequency distribution of fluctuations of the intensity of laser light scattered from the liquid mixture of a sample and LAL changes with growth of the gel particles in the liquid mixture. Then, a change in profile of the frequency distribution of fluctuations of the intensity of light scattered from the liquid mixture of a sample and LAL makes it possible to detect the aggregation-starting time of the reaction system.

Therefore, according to the present invention, the growth process of gel particles can be detected with higher accuracy by detecting the distribution of particle size of gel particles in the liquid mixture of a sample and LAL almost in real time. As a result, the aggregation-starting time of the reaction system can be detected with higher accuracy or more promptly. Then, a relationship between the aggregation-starting time of the reaction system and the concentration of a predetermined biologically active substance is investigated in advance. Consequently, it becomes possible to measure the predetermined biologically active substance in the liquid mixture of the sample and LAL more accurately or more quickly.

In the present invention, the auto-correlation function of fluctuations of the scattered light intensity may be obtained to detect the aggregation-starting time of the liquid mixture based on a change in profile of the auto-correlation function over time.

A frequency distribution having a stronger contrast can be obtained by obtaining the auto-correlation function of fluctuations of the intensity of light scattered from the liquid mixture of LAL and a sample containing a predetermined biologically active substance. Therefore, the generation and growth of gel particles can be detected in the reaction system of the predetermined biologically active substance and LAL with higher accuracy.

The inventors have intensively studied and found that the auto-correlation function of fluctuations of the intensity of light scattered from a liquid mixture of LAL and a sample containing a predetermined biologically active substance varies with the passage of time after the start of reaction. In other words, the initial stage after the reaction start has totally an auto-correlation function of almost 1 regardless of the value of the delay time of the auto-correlation function and causes pulsation in a region with a comparatively short delay time (hereinafter, also referred to as a "short-delay time region").

After that, the auto-correlation function in a region with a longer delay time compared with that of the short-delay time region (hereinafter, referred to as a "long-delay time region") is once reduced with the passage of time. When time passes more, the pulsation found in the short-delay time region disappears and pulsation appears in the long-delay time region. The auto-correlation coefficient in the long-delay time region (hereinafter, "DC component" of the auto-correlation function) increases.

Here, the pulsation found in the short-delay time region at the initial stage after the reaction start means the presence of gel particles with comparatively small diameters. Furthermore, that the pulsation found in the short-delay time region disappears with the passage of time and pulsation appears in the long-delay time region means that the diameters of gel particles increase. Furthermore, the phenomenon of an increase in auto-correlation coefficient (DC component) in the long-delay time region means that protein denaturation and gel network are progressing.

Thus, there is a close relationship between a change in profile of auto-correlation function over time and the growth process of gel particles in the liquid mixture. Therefore, the auto-correlation function of fluctuations of scattered light intensity is acquired, and the aggregation-starting time in the reaction system of a predetermined biologically active substance and LAL is detected based on a change in profile of auto-correlation function over time. As a result, the aggregation-starting time can be detected with higher accuracy.

In the present invention, in the profile of the auto-correlation function, a time at which pulsation disappears in a predetermined region with a short delay time may be used as the aggregation-starting time. Here, the predetermined region with a short delay time may be the above short-delay time region. In this case, a time at which gel particles with small diameters disappear may be set as an aggregation-starting time. Thus, the aggregation-starting time in a reaction system of a predetermined biologically active substance and LAL can be detected more easily or more promptly.

Also in the present invention, in the profile of the auto-correlation function, a time at which pulsation disappears in a predetermined region with a short delay time and pulsation appears in a region with a longer delay time than the predetermined region may be used as the aggregation-starting time. Here, a region with a longer delay time compared with that of the above predetermined region may be the above long-delay time region. In this case, an aggregation-starting time can be detected at a time at which gel particles with small diameters disappear and the gel particles aggregate and start becominglarge. Thus, the aggregation-start time, which is a time at which the aggregation is initiated, can be detected with higher accuracy.

Furthermore, the above predetermined region may be a region with a delay time of 1 msec or less. In the actual auto-correlation function in a reaction system of a predetermined biologically active substance and LAL, in many cases, pulsation is found in a region with a delay time of 1 msec or less in the initial stage of the reaction. Thus, with the passage of a reaction time, pulsation in the region with a delay time of 1 msec or less disappears. Then, in many cases, pulsation is initiated in a region with a delay time of more than 1 msec. Therefore, when the above predetermined region may be a region with a delay time of 1 msec or less, an aggregation-starting time can be detected with higher accuracy in reality.

Also in the present invention, a time at which a relaxation coefficient of the auto-correlation function once decreases with the passage of time and then increases may be used as the aggregation-starting time.

When the relaxation coefficient of the auto-correlation function has once decreased with the passage of time and then increased again, DC component signals in a region with a delay time longer than that of the predetermined region increase and the protein denaturation and gel network may be progressing. Therefore, a time at which the relaxation coefficient of the auto-correlation function once decreases with the passage of time and then increases may be set to the above aggregation-starting time. In this case, a time at which protein denaturation and gel network begin to advance can be regarded as an aggregation-starting time, and the aggregation-starting time can be detected with higher accuracy.

Also in the present invention, a time at which a relaxation coefficient of the auto-correlation function once decreases to the minimum with the passage of time and then increases may be used as the aggregation-starting time.

It is found that a process in which the relaxation coefficient of the auto-correlation function decreases first with the passage of time and a process in which pulsation disappears in the above predetermined region are substantially synchronized. Furthermore, as described above, when the relaxation coefficient of the auto-correlation function has once decreased with the passage of time and then increased again, DC component signals in a region with a delay time longer than that of the predetermined time increase, and it indicates that the protein denaturation and gel network are progressing.

When the above aggregation-starting time is set to a time at which the relaxation coefficient in profile of the auto-correlation function once decreases to the minimum with the passage of time and then increases, the moment at which the gel particles of small particle size disappear and gel particles of large particle size begin to increase, and the protein denaturation and gel network do not still progress, can be detected. Thus, the aggregation-starting time in a reaction system of a predetermined biologically active substance and LAL can be detected more accurately.

In the present invention, furthermore, the above liquid mixture may be stirred to obtain the above scattered light intensity. The growth of gel particles can be accelerated as particles by stirring. In addition, the stirringmakes the gel particles uniformly distribute in the liquid mixture. Thus, an aggregation-starting time can be detected with higher accuracy.

For example, the biogenous biologically active substance may be endotoxin or β-D-glucan. If the present invention is applied to endotoxin or β-D-glucan, the detection or concentration measurement of endotoxin, which is the most typical pyrogen, can be performed more correctly. Infusion solutions, injection agents, the blood, or the like, which are contaminated with endotoxin, can be prevented from being introduced into the human body and causing side effects. Similarly, detection or concentration measurement of β-D-glucan can be also performed more correctly. Thus, it is possible to screen fungal infections of a wide variety of fungi including not only those commonly found in clinical sites, such as Candida, Spergillus, and Cryptococcus, but also uncommon fungi.

The present invention may be an apparatus for measuring a biogenous biologically active substance by reacting a biogenous biologically active substance in a sample with a limulus amoebocyte lysate, LAL, and detecting the biologically active substance in the sample or measuring the concentration of the biologically active substance, the apparatus including:
a liquid mixture retaining means for retaining a liquid mixture of the sample and LAL while allowing light to be incident on the liquid mixture, and allowing a reaction of the biologically active substance with LAL to progress;
a light incidence means for entering light into the liquid mixture in the liquid mixture retaining means;
a stirring means for stirring the liquid mixture in the liquid mixture retaining means;
a light receiving means for receiving scattered light of the incident light on the liquid mixture and converting the scattered light into an electric signal;
an aggregation-starting time detecting means for detecting an aggregation-starting time in the liquid mixture based on a temporal change of frequency distribution of fluctuations of the intensity of the scattered light, which is obtained from the electric signal converted in the light receiving means; and
a deriving means for detecting the biologically active substance in the sample or deriving the concentration of the biologically active substance in the sample by means of the aggregation-starting time detected by the aggregation-starting time detecting means.

In the apparatus for measuring a biogenous biologically active substance according to the present invention, the aggregation-starting time detecting means may obtain an auto-correlation function of fluctuations of the intensity of the scattered light, and
detect an aggregation-starting time in the liquid mixture based on a temporal change in profile of the auto-correlation function.

In the apparatus for measuring a biogenous biologically active substance according to the present invention,
the aggregation-starting time detecting means may use, in the profile of the auto-correlation function, a time at which pulsation disappears in a predetermined region with a short delay time as the aggregation-starting time.
Also, in the profile of the auto-correlation function, a time at which pulsation disappears in a predetermined region with a short delay time and pulsation appears in a region with a longer delay time than the predetermined region may be used as the aggregation-starting time.
The predetermined region may be a region with a delay time of 1 msec or less.

In the apparatus for measuring a biogenous biologically active substance according to the present invention,
the aggregation-starting time detecting means may use a time at which a relaxation coefficient of the auto-correlation function once decreases with the passage of time and then increases as the aggregation-starting time. Also, a time at which a relaxation coefficient of the auto-correlation function once decreases to the minimum with the passage of time and then increases may be used as the aggregation-starting time.

In the apparatus for measuring a biogenous biologically active substance according to the present invention, the biogenous biologically active substance may be endotoxin or β-D-glucan.

Here, the above means for attaining the objects of the present invention may be used in combination as much as possible.

### ADVANTAGEOUS EFFECTS OF INVENTION

The present invention enables higher measurement accuracy or shortened measurement time to be achieved when using the reaction between LAL and a biogenous biologically active substance, such as endotoxin and β-D-glucan, to detect or to measure the concentration of the biologically active substance.

### BRIEF DESCRIPTION OF THE DRAWINGS

FIG. 1 is a diagram illustrating a schematic configuration of a measurement system of a predetermined biologically active substance in an example of the present invention.
FIG. 2 is a diagram illustrating a temporal change in an auto-correlation function of fluctuations of scattered light intensity obtained in the example of the present invention.
FIG. 3 is a graph illustrating a temporal change in relaxation coefficient of the auto-correlation function of fluctuations of scattered light intensity obtained in the example of a the present invention.
FIG. 4 is a graph illustrating the results of a comparison between the example of the present invention and the conventional method with respect to the relationship between an aggregation-starting time and the concentration of endotoxin.
FIG. 5 is a schematic diagram illustrating a process where endotoxin or β-D-glucan causes LAL to turn into a gel and a method for detecting the gelation.

### BEST MODE FOR CARRYING OUT THE INVENTION

### <Example 1>

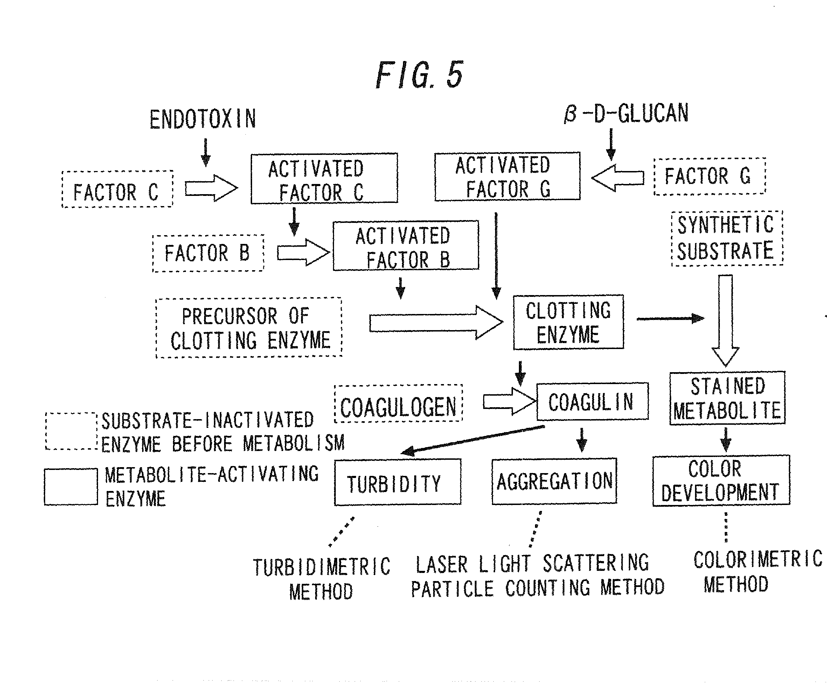

A process of forming a gel by a reaction of LAL with endotoxin has been well studied. In other words, as illustrated in FIG. 5, when endotoxin binds to Factor C, which is serine protease in LAL, the Factor C is activated and changed to activated Factor C. The activated Factor C hydrolyzes and activates Factor B, which is another serine protease in LAL, to obtain an activated Factor B. The activated Factor B directly hydrolyzes precursor of clotting enzyme in LAL to obtain clotting enzyme. Furthermore, the clotting enzyme hydrolyzes coagulogen in LAL to generate coagulin. Then, the generated coagulins are assembled together to further generate an insoluble gel. As a result, the entire LAL is considered to be involved in this gelation and turn into a gel.

Likewise, when β-D-glucan binds to Factor G in LAL, the Factor G is activated and turned into activated Factor G. Then, the activated Factor G hydrolyzes precursor of clotting enzyme in LAL to generate clotting enzyme. As a result, in a manner similar to the reaction between endotoxin and LAL, coagulin is generated. Then, the generated coagulins are assembled together to further generate an insoluble gel.

This series of reactions is similar to the generation process of fibrin gels through serine proteases for Christmas factors and thrombins observed in mammals. Even if the amount of an activator is very small in such an enzyme cascade reaction, it has very strong amplification function to consecutively activate subsequent cascades. Therefore, according to the method for measuring the predetermined biologically active substance using LAL, it is possible to detect an extremely small amount of the predetermined biologically active substance in the order of sub-picogram per milliliter.

Measurement methods, which are capable of quantifying predetermined biologically active substances include the turbidimetric method and the light scattering method (laser light scattering particle counting method) as described above. As illustrated in FIG. 5, any of these measurement methods detects an aggregate of coagulins generated by its LAL enzyme cascade reaction as the turbidity of a sample in the case of the former and as gel fine particles generated in the system in the case of the latter. Thus, a high-sensitive measurement can be performed.

Particularly, the light scattering method directly measures fine particles in a gel generated in the system. Thus, the sensitivity of the light scattering method is higher than that of the turbidimetric method. In addition, the light scatteringmethodstirs compulsorily the sample, which generally contains LAL and an analyte. Therefore, the light scattering method can detect the generation of gel within a short time as compared with the turbidimetric method.

Another method for endotoxin measurement is a colorimetric method. As illustrated in FIG. 5, the colorimetric method utilizes a LAL enzyme cascade reaction but does not measure the turbidity of a sample by a coagulin gel. The colorimetric method is a method for measuring a change in absorbance of a sample by using a synthetic substrate which can be hydrolyzed by coagulase and colored and reacting an analyte with LAL containing the synthetic substrate. This colorimetric method measures the concentration of a coloring substance generated in the system. Thus, the colorimetric method can measure a low-concentration predetermined biologically active substance within a short time as compared with the turbidimetric method and light scattering method which measure the generation of gel in a sample.

Unlike the colorimetric method, the turbidimetric method has been evaluated as a convenient method at the work site in that no special reagent is required, a wide range of concentration of a predetermined biologically active substance is measurable, and so on. On the other hand, however, the turbidimetric method has a disadvantage in that an extremely long time is required for measurement of a predetermined biologically active substance in low concentration. This is because the turbidimetric method does not directly focus on the amount of coagulin produced, which is the final product of a protease cascade, but focuses on a process in which optical transmittance decreases as a gel is subsequently formed by association of coagulin.

In other words, gelation does not occur until the concentration of coagulin reaches a certain level or more, so that detection of a predetermined biologically active substance in the turbidimetric method should wait for the formation of a gel. Therefore, the coagulin in necessary and sufficient concentration can be quickly generated and turn into a gel when the concentration of the predetermined biologically active substance is high. Thus, the measurement time can be shortened. In contrast, when the concentration of the predetermined biologically active substance is low, it takes a long time to reach the concentration of coagulin necessary for gelation. Thus, the measurement time can be prolonged.

The light scattering method has improved points in comparison with the turbidimetric method. One of the improved points is to stir a sample and the other thereof is to detect particles by incident light, such as laser light, but not direct gelation. Thus, the light scattering method can significantly shorten the measurement time, compared with the turbidimetricmethod. However, the gel particles observed are comparatively large (several micrometers or more). Thus, the degree of shortening a measurement time has been less than that of the colorimetric method. The turbidimetric method and the light scattering method are common in that a time at which a physical quantity exceeds a certain constant threshold is regarded as a reaction start point even though these methods focus on different physical quantities.

On the other hand, the above colorimetric method detects color development of a stained metabolite of a synthetic substrate which is equivalent to the end product of a protease cascade. Thus, the colorimetric methodmaydetect the degree of progress (rate of increase = differentiation) of color development within a predetermined time. Therefore, since it is not necessary to wait until gelation occurs, a measurement time can be shortened. However, there are problems in that, for example, a special reagent is required and a measurable concentration range is narrow.

In the present invention, to solve disadvantages of the various methods as described above, the present inventors have completed a method for shortening a measurement time in a light scattering method by application of a dynamic light-scattering measurement technique to the light scattering method. Hereinafter, embodiments of the present invention will be exemplarily described in detail with reference to the attached drawings. However, the present invention is not limited to the embodiments described below.

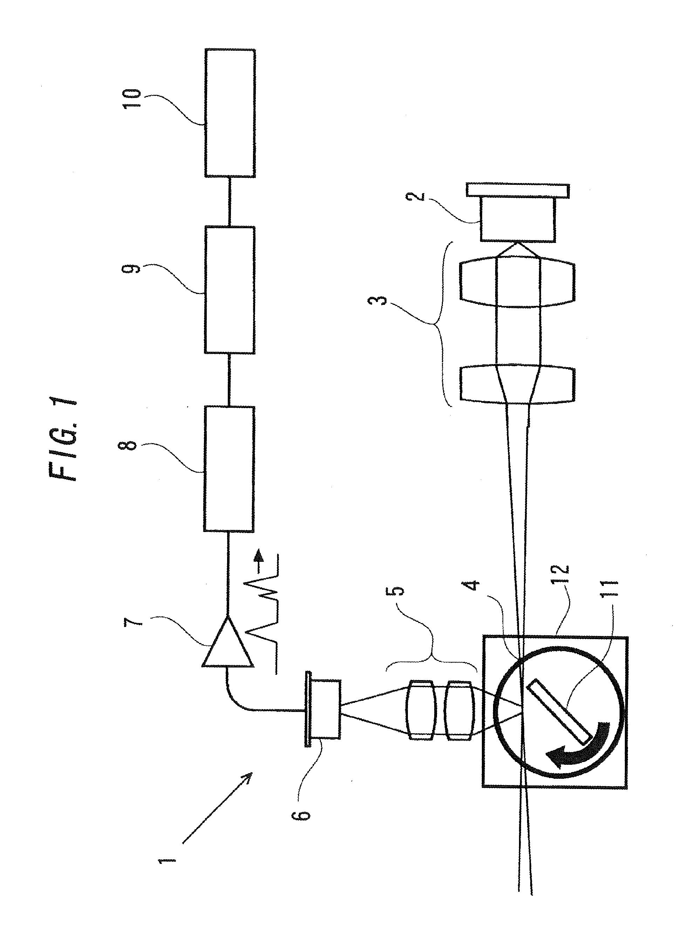

FIG. 1 is a diagram illustrating a schematic configuration of a measurement system 1 for a predetermined biologically active substance (endotoxin in this example) in the present example. A light source 2 used in the measurement system 1 is a laser, a super high-intensity LED, or the like. Light irradiated from the light source 2 is concentrated by an incidence optical system 3 and then incident on a sample cell 4. The sample cell 4 retains a liquid mixture of a sample for measurement of a predetermined biologically active substance and a LAL reagent. Light incident on the sample cell 4 is scattered from gel particles in the liquid mixture. A stirring bar 11 is placed in the sample cell 4. The liquid mixture in the sample cell 4 is stirred by rotation of the stirring bar 11 caused by action of a variable magnetic field which is applied from the outside by a magnetic stirrer 12.

An output optical system 5 is arranged on the lateral side of an incident optical axis in the sample cell 4. In addition, a light receiving element 6 is arranged on the extension of the optical axis of the output optical system 5. Here, the light receiving element 6 is provided for receiving scattered light, which is scattered by particles in the liquid mixture in the sample cell 4 and concentrated by the output optical system 5, and converting the received light into an electric signal. The light receiving element 6 is electrically connected to an amplifying circuit 7 for amplifying the electric signal photoelectrically converted by the light receiving element 6; a filter 8 for removing a noise from the electric signal amplified by the amplifying circuit 7; a correlator 9 for calculating an auto-correlation function of scattered light from the electric signal after the noise was removed; and an arithmetic unit 10 which derives an aggregation-starting time and derives the concentration of a predetermined biologically active substance based on a temporal change in profile of the auto-correlation function of scattered light. Furthermore, a display unit (not illustrated) for displaying calculation results may be further connected.

The measurement system 1 illustrated in FIG. 1 constitutes an apparatus for measuring a biogenous biologically active substance in this example. In the measurement system 1, the sample cell 4 is equivalent to a liquid mixture retaining means. The light source 2 and the incidence optical system 3 constitute a light incidence means. The stirring bar 11 and the magnetic stirrer 12 constitute a stirring means. The output optical system 5 and the light receiving element 6 constitute a light receiving means. Parts of the correlator 9 and the arithmetic unit 10, which are responsible for deriving an aggregation-starting time, constitute an aggregation-starting time detecting means. The arithmetic unit 10 has a part for deriving the concentration of a predetermined biologically active substance from an aggregation-starting time. This part is equivalent to a deriving means. In this example, the stirring means includes the stirring bar 11 and the magnetic stirrer 12. Alternatively, the stirring means may be configured so that an ultrasonic vibrator or the like vibrates a liquidmixture to stir the liquidmixture.

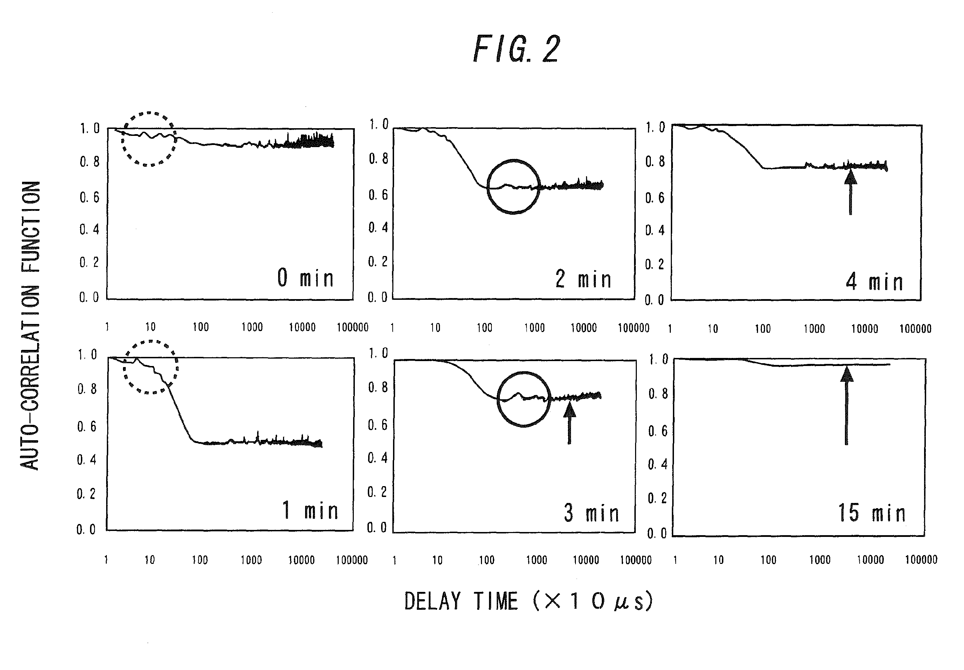

Next, in the above measurement system 1, a measurement is performed on a liquid mixture in which LAL is mixed with a sample containing endotoxin. Then, from the resulting light-scattering signals of time-series, a time-resolved auto-correlation function is calculated and the results are illustrated in FIG. 2. Six graphs in FIG. 2 are auto-correlation functions at the start of a reaction, and 1, 2, 3, 4, and 15 minutes after the start of the reaction when a sample containing a comparatively high concentration of endotoxin (0.6 EU/mL) is mixed with a LAL reagent, respectively. In each graph, the vertical axis represents the value of the auto-correlation coefficient and the horizontal axis represents the value of the delay time. Note that the aggregation-starting time of the liquid mixture was 6.5 minutes when measured by a static light-scattering method.

As is evident from FIG. 2, in any of graphs of different elapsed times, there is a tendency that the auto-correction coefficient once decreases with an increase in elapsed time and subsequently it keeps almost a constant value. This tendency can be observed in the present measurement system itself and may be referred to as an instrumental function. When the reaction system of endotoxin and LAL is measured in the present measurement system, pulsation is observed on a curve at a region with a delay time of 1 msec or less (this region is referred to as a "predetermined region with a short delay time = short-delay time region in the present example) in the state of 0 (zero) minute in delay time (dashed line of circle in the figure). This is due to a large number of components of comparatively high frequencies in fluctuations of the scattered light intensity. This fact indicates that a large number of particles of comparatively small diameters are in the reaction system.

Subsequently, when the time elapses 1 minute from the start of the reaction, a phenomenon in which the auto-correlation coefficient at a region with a delay time of longer than 1 msec (this region is referred to as a "long-delay time region" in the present embodiment) is entirely decreased at one minute elapsed from the start of the reaction. In this case, however, pulsation is also observed on the short-delay time region. Then, pulsation is observed in the long-delay time region after 2 minutes elapsed from the start of the reaction. After 3 minutes from the start of the reaction, pulsation in the short-delay time region disappears, and the pulsation in the long-delay time region becomes large. This is due to the fact that components in fluctuations of the intensity of light scattered from the liquid mixture are shifted from the high-frequency side to the low-frequency side with the time elapsed from the start of reaction. Thus, it represents that the state is shifted from one with a large number of gel particles with small diameters to one with a large number of gel particles with large diameters in the liquid mixture.

Furthermore, when the time elapsed from the reaction start is 2 minutes or more, it is observed that the damping time of an auto-correlation coefficient gradually becomes long with an increase in elapsed time and the DC component increases on the long-delay time side of a region where the auto-correlation coefficient decreases rapidly. It is considered due to the progress of protein denaturation and gel network in the liquid mixture.

From the above findings, in the present example, an aggregation-starting time of the present reaction system is a time at which pulsation disappears from the short-delay time region and pulsation begins to sparsely appear on the long-delay time region during the change in profile of the above auto-correlation function. This time is a time at which the liquid mixture enters a state where gel particles of small diameters disappear and gel particles of large diameters begin to appear but sparsely. In other words, the time is the moment at which gel particles of small diameters are aggregated and begin to increase in size. Here, the term "moment" means a time period of about 30 to 60 seconds.

Here, in the case of a gelation-reaction system model of a liquid mixture of LAL and an endotoxin-containing sample, which is known at present, there is a phase on the initial stage of the reaction after the start thereof. In this phase, the nucleus of the gelation reaction is formed. This nucleus includes particles of small diameters, such as coagulogen, coagulin monomers, and small oligomers.

Subsequently, the reaction goes into a gelation initial phase where any of long rods and long random coils is formed from the above nucleus. After further elapsing time, the fraction of long rods in the liquid mixture decreases, while the fraction of the long random coils, the reflective species, and proteins increases. This kind of change in the fractions leads to an increase in turbidity.

When the time elapses further, coagulin monomers directly interact with the gel network and are then incorporated into the gel network as a part thereof. Thus, it is transferred to the transition phase of the gel.

At present, the above "particles of small diameters" are considered to correspond to small particles, coagulogen, coagulin monomers, and small oligomers in the reaction system model of endotoxin and LAL. The above "particles of large diameters" are considered to correspond to long rods and long random coils, and "DC components" are considered to correspond to the reflective species and proteins.

### <Example 2>

Next, Example 2 of the present invention will be described. An aggregation-starting time in the present example is detected as a time at which a relaxation coefficient behaves such that it becomes the minimum when the relaxation coefficient of an auto-correlation coefficient decreases rapidly and then increases. A measurement system in the present example is equivalent to the measurement system 1 illustrated in FIG. 1.

### [Experiment]

Reference standard endotoxin was dissolved in Five milliliters of LAL reagent water (hereinafter, also referred to as "LRW") to prepare an endotoxin solution of 2000 EU/mL in volume as a stock solution. Then, the stock solution was further dissolved with LRW to prepare dilution series of 0.001 to 100 EU/mL. Here, the LAL reagent used was Limulus ES-I Single Test Wako (manufactured by Wako Pure Chemical Industries, Ltd.).

### [Measurement pretreatment]

An endotoxin sample (200 µL) was added to a glass tube with ES-II (LAL reagent) and then stirred for 5 seconds by a vortex. Immediately after that, the sample was transferred into a glass sample cell (cuvette) of 7 mm in diameter and then subj ected to a measurement using the measurement system 1. Furthermore, the sample was previously subjected to sterilization by heat at 250°C for 2 hours or more.

### [Results]

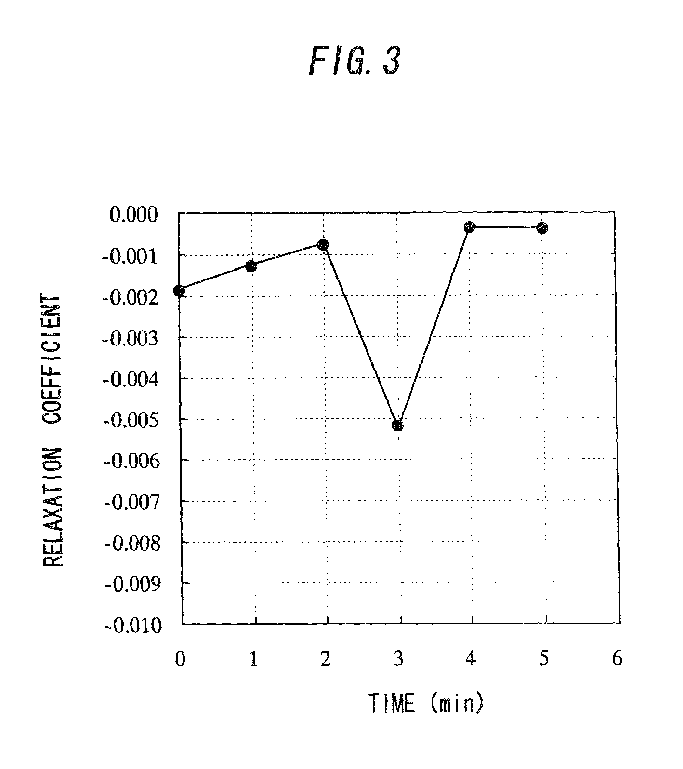

Results obtained by the measurement of a temporal change in relaxation coefficient with respect to the auto-correlation function of fluctuations of scattered light intensity are illustrated in FIG. 3. Here, the relaxation coefficient represents the inclination of a decreasing portion of the auto-correlation function when the horizontal axis of the graph of the auto-correlation function, such as one illustrated in FIG. 2, is not provided as a logarithmic axis but provided as a linear axis. As is evident from FIG. 3, the relaxation time increases with the passage of time after the reaction start, but it rapidly decreases at a certain time. The above measurement was performed on various samples of different endotoxin concentrations. It is found that a discontinuous decrease in this relaxation coefficient certainly occurs without depending on the endotoxin concentration in the measurement sample. Furthermore, it is found that a time until this phenomenon occurs after the start of a reaction between endotoxin and LAL depends on the endotoxin concentration in the sample. In the present example, a time at which the relaxation coefficient rapidly decreases (time at which the relaxation coefficient becomes the minimum) is regarded as an aggregation-starting time.

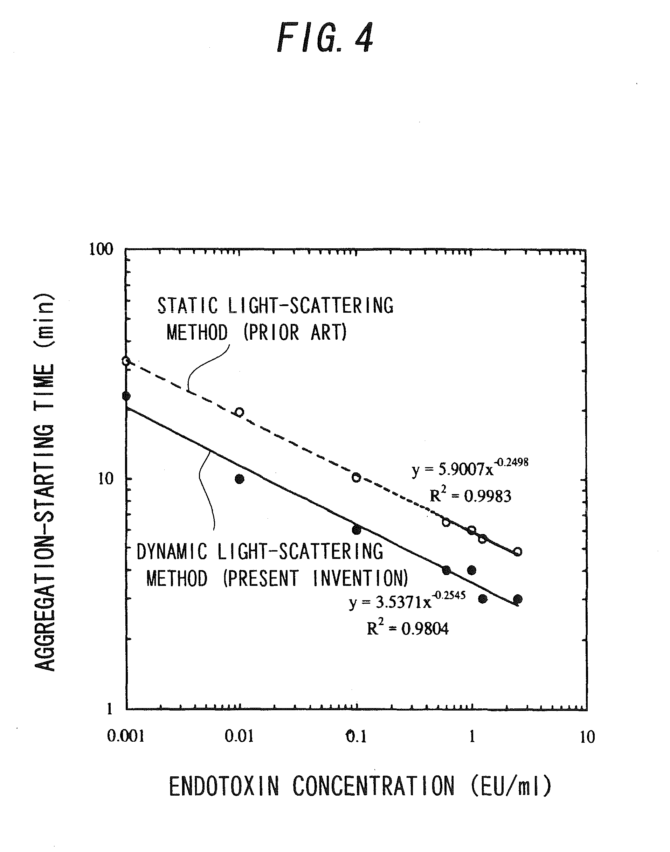

The aggregation-starting time measured in this example was plotted with respect to the endotoxin concentration. The results are illustrated in FIG. 4. In FIG. 4, a white circle represents an aggregation-starting time measured by the conventional (static) light-scattering method. On the other hand, a black circle represents the aggregation-starting time obtained by the method of the present example. From the figure, the time required for detection of an aggregation-starting time by the method of the present example is almost half shorter than one by the method where the measurement is performed using the conventional static light scattering method. From these results, the advancing state of the reaction of endotoxin and LAL is regarded as a change in profile of the auto-correlation function to enable the endotoxin measurement to be performed within a shorter time.

Conventionally, in the measurement using the turbidimetricmethod, the aggregation-starting time has been set to a time at which the transmittance of a liquid mixture of a LAL reagent and an endotoxin-containing sample decreases from 100% to 95% immediately after the start of the measurement. Furthermore, in the (static) light scattering method, an aggregation-starting time has been set to a time at which, for example, 20 or more aggregated clusters are obtained. However, the above values of 95% and 20 have been artificially determined on the ground of a measurement personnel. Thus, these values have unclear physical meaning. In contrast, in the method of the present example in which the aggregation-starting time is set to a time at which the relaxation coefficient of the auto-correlation function decreases, the aggregation-starting time has an interpretive physical meaning of the moment prior to an increase in DC component, that is, the moment before the progression of protein denaturation and gel network in the reaction system of endotoxin and LAL.

Furthermore, according to the method of the present example as described above, it is confirmed that the measurement can be performed in time almost half of the aggregation-starting time of the (static) light scattering method. Furthermore, in the method of the present example, it is possible to increase the number of scattered light data by extending a measurement region by means of enlarging the diameter of laser light or the like without increasing the measurement time (processable with ensemble mean but not with time average). Furthermore, even if the measurement region is extended, it has a small risk of causing a decrease in S/N ratio unlike the conventional (static) light scattering method. Therefore, in the method of the present invention, the expansion of the measurement region, which is undesirable in the conventional (static) light scattering method, can be effectively used for speeding up the measurement and increasing the sensitivity thereof.

In Example 2, furthermore, a time at which the relaxation coefficient rapidly decreases (time at which the relaxation coefficient becomes the minimum) is regarded as an aggregation-starting time. Alternatively, the aggregation-starting time may be a time at which the relaxation coefficient is not the minimum as long as it is within a time period from the beginning of a rapid decreases in relaxation coefficient to the recovery thereof. Such an aggregation-stating time may also lead to a sufficiently accurate or quick detection of the onset of aggregation. In the present example, the above time period from the beginning of a rapid decreases in relaxation coefficient to the recovery thereof is equivalent to a time when the relaxation coefficient increases after once decreased with time.

Furthermore, in the above example, an auto-correlation function was obtained to obtain a temporal change in frequency distribution in fluctuations of the intensity of light scattered from the reaction system of endotoxin and LAL. However, the present invention does not always need to obtain an auto-correlation function of frequency distribution in fluctuations of the scattered light intensity. Since the tendency which appears in a temporal change in profile of the auto-correlation function should appear on any graphical representation of the frequency distribution. Thus, the aggregation-starting time can be obtained with high accuracy or quickly by application of the method equivalent to that of the present example as described above.

Furthermore, in the above examples, the descriptions have been made on the measurement of endotoxin as an example of the predetermined biologically active substance. In the above examples, any of other predetermined biologically active substances, such as β-D-glucan, may be applicable.

### DESCRIPTION OF REFERENCE NUMERALS

- 1: measurement system
- 2: light source
- 3: incidence optical system
- 4: sample cell
- 5: output optical system
- 6: light receiving element
- 7: amplifying circuit
- 8: denoising filter
- 9: correlator
- 10: arithmetic unit
- 11: stirring bar
- 12: magnetic stirrer

## Claims

1. A method for measuring a biologically active substance of biological origin, including reacting a biologically active substance of biological origin in a sample with a limulus amoebocyte lysate, LAL, and detecting the biologically active substance in the sample or measuring the concentration of the biologically active substance, the method, **characterized in that**:
after mixing the sample and LAL, making light incident on a liquid mixture of the sample and LAL;
obtaining an intensity of scattered light of the incident light from the liquid mixture;
detecting an aggregation-starting time in the liquid mixture based on a temporal change of frequency distribution of fluctuations of the intensity of the scattered light; and
detecting the biologically active substance in the sample or measuring the concentration of the biologically active substance in the sample by means of the aggregation-starting time.

2. The method for measuring a biologically active substance of biological origin according to claim 1, **characterized in that**:
obtaining an auto-correlation function of fluctuations of the intensity of the scattered light; and
detecting an aggregation-starting time in the liquid mixture based on a temporal change in profile of the auto-correlation function.

3. The method for measuring a biologically active substance of biological origin according to claim 2, wherein
in the profile of the auto-correlation function, a time at which pulsation disappears in a predetermined region with a short delay time is used as the aggregation-starting time.

4. The method for measuring a biologically active substance of biological origin according to claim 2, wherein
in the profile of the auto-correlation function, a time at which pulsation disappears in a predetermined region with a short delay time and pulsation appears in a region with a longer delay time than the predetermined region is used as the aggregation-starting time.

5. The method for measuring a biologically active substance of biological origin according to claim 3 or 4, wherein
the predetermined region is a region with a delay time of 1 msec or less.

6. The method for measuring a biologically active substance of biological origin according to claim 2, wherein
a time at which a relaxation coefficient of the auto-correlation function once decreases with the passage of time and then increases is used as the aggregation-starting time.

7. The method for measuring a biologically active substance of biological origin according to claim 6, wherein
a time at which a relaxation coefficient of the auto-correlation function once decreases to the minimum with the passage of time and then increases is used as the aggregation-starting time.

8. The method for measuring a biologically active substance of biological origin according to any of claims 1 to 7, wherein
the liquid mixture is stirred when the intensity of the scattered light is obtained.

9. The method for measuring a biologically active substance of biological origin according to any of claims 1 to 8, wherein
the biologically active substance of biological origin is endotoxin or β-D-glucan.

10. An apparatus for measuring a biologically active substance of biological origin by reacting a biologically active substance of biological origin in a sample with a limulus amoebocyte lysate, LAL, and detecting the biologically active substance in the sample or measuring the concentration of the biologically active substance, the apparatus comprising:
a liquid mixture retaining means for retaining a liquid mixture of the sample and LAL while allowing light to be incident on the liquid mixture, and allowing a reaction of the biologically active substance with LAL to progress;
a light incidence means for entering light into the liquidmixture in the liquid mixture retaining means;
a stirring means for stirring the liquid mixture in the liquid mixture retaining means;
a light receiving means for receiving scattered light of the incident light on the liquid mixture and converting the scattered light into an electric signal;
an aggregation-starting time detecting means for detecting an aggregation-starting time in the liquid mixture based on a temporal change of frequency distribution of fluctuations of the intensity of the scattered light, which is obtained from the electric signal converted in the light receiving means; and
a deriving means for detecting the biologically active substance in the sample or deriving the concentration of the biologically active substance in the sample by means of the aggregation-starting time detected by the aggregation-starting time detecting means.

11. The apparatus for measuring a biologically active substance of biological origin according to claim 10, wherein
the aggregation-starting time detecting means obtains an auto-correlation function of fluctuations of the intensity of the scattered light, and
detects an aggregation-starting time in the liquid mixture based on a temporal change in profile of the auto-correlation function.

12. The apparatus for measuring a biologically active substance of biological origin according to claim 11, wherein
theaggregation-startingtimedetecting means uses, in the profile of the auto-correlation function, a time at which pulsation disappears in a predetermined region with a short delay time as the aggregation-starting time.

13. The apparatus for measuring a biologically active substance of biological origin according to claim 11, wherein
theaggregation-startingtimedetectingmeansuses, in the profile of the auto-correlation function, a time at which pulsation disappears in a predetermined region with a short delay time and pulsation appears in a region with a longer delay time than the predetermined region as the aggregation-starting time.

14. The apparatus for measuring a biologically active substance of biological origin according to claim 12 or 13, wherein
the predetermined region is a region with a delay time of 1 msec or less.

15. The apparatus for measuring a biologically active substance of biological origin according to claim 11, wherein
theaggregation-startingtimedetectingmeansuses a time at which a relaxation coefficient of the auto-correlation function once decreases with the passage of time and then increases as the aggregation-starting time.

16. The apparatus for measuring a biologically active substance of biological origin according to claim 11, wherein
theaggregation-startingtimedetectingmeansuses a time at which a relaxation coefficient of the auto-correlation function once decreases to the minimum with the passage of time and then increases as the aggregation-starting time.

17. The apparatus for measuring a biologically active substance of biological origin according to any one of claims 10 to 16, wherein
the biologically active substance of biological origin is endotoxin or β-D-glucan.
